(19) Europäisches Patentamt
European Patent Office
Office européen des brevets

(11) Numéro de publication : **0 434 576 A1**

(12) **DEMANDE DE BREVET EUROPEEN**

(21) Numéro de dépôt : **90420534.1**

(22) Date de dépôt : **11.12.90**

(51) Int. Cl.[5] : **C07D 239/54**, C07D 213/75, C09K 19/34

(30) Priorité : **13.12.89 FR 8916751**

(43) Date de publication de la demande : **26.06.91 Bulletin 91/26**

(84) Etats contractants désignés : **BE DE FR GB IT NL SE**

(71) Demandeur : **RHONE-POULENC CHIMIE
25, quai Paul Doumer
F-92408 Courbevoie Cédex (FR)**

(72) Inventeur : **Brienne, Marie-Josèphe
10, rue Suger
F-75006 Paris (FR)**
Inventeur : **Lehn, Jean-Marie
21, rue d'Oslo
F-67000 Strasbourg (FR)**
Inventeur : **Stibor, Ivan, Prague Institute of Chem. Technology
Department of Organic Chemistry, Technicka 5
166 28 Prague 6 (CS)**

(74) Mandataire : **Trolliet, Maurice et al
RHONE-POULENC INTERSERVICES Service Brevets Chimie Centre de Recherches des Carrières B.P. 62
F-69192 Saint-Fons Cédex (FR)**

(54) **Adducts thermotropes à partir d'espèces non mésogènes liées par liaisons hydrogène et leur procédé de préparation.**

(57) La présente invention concerne des adducts thermotropes répondant à la formule générale : $(A)_p\!-\!\!-\Sigma\ ---\ \bar{\Sigma}\!-\!\!-(B)_q$ dans laquelle : $\Sigma$ représente un groupe formant au moins deux liaisons hydrogène avec le groupe fonctionnel complémentaire représenté par le symbole $\bar{\Sigma}$ ; A et B représentent des substitutions appropriées, portées par les groupes fonctionnels $\Sigma$ et $\bar{\Sigma}$, qui contribuent en combinaison avec la structure du motif central $\Sigma$ — $\bar{\Sigma}$ à induire la propriété thermotrope ; p et q = 1 ou 2. Le motif central $\Sigma$ — $\bar{\Sigma}$ dérive notamment des couples complémentaires : cytosine/isocytosine, guanine/cytosine, adénine/uracil, dialkanamido-2,6 pyridine/uracil, dialkanamido-2,6 pyridine/thymine. Les substituants A et B sont notamment de longues chaînes aliphatiques hydrocarbonées, pouvant renfermer un ou plusieurs hétéroatomes, qui sont ramifiées et possèdent au moins 28 atomes.

EP 0 434 576 A1

# ADDUCTS THERMOTROPES A PARTIR D'ESPECES NON MESOGENES LIEES PAR LIAISONS HYDROGENE ET LEUR PROCEDE DE PREPARATION

La présente invention concerne des adducts thermotropes formés à partir de deux espèces non mésogènes de structures différentes liées entre elles par au moins deux liaisons hydrogène.

Dans le but de disposer de matériaux présentant des propriétés physiques intéressantes, notamment du matériaux capables de présenter un degré d'organisation les rendant utilisables notamment pour la réalisation de composants optiques solides, de polariseurs linéaires ou circulaires, de matériaux à forte non linéarité optique, la Demanderesse a mis au point la synthèse et a étudié les caractéristiques physico-chimiques de nouveaux matériaux thermotropes ayant la structure particulière d'adduct énoncée ci-avant. Il est connu dans l'art antérieur (cf. T. KATO et al., J. Am. Chem. Soc. 1989, 111, 8533-8534) d'associer par l'intermédiaire d'une liaison hydrogène deux espèces mésogènes proprement dites et il en résulte un élargissement de l'étendue en températures de la plage d'anisotropie par rapport à ce qui se passe avec les espèces mésogènes de départ prises séparément ; mais on ne dit rien dans cet art antérieur à propos de la possibilité de synthétiser, comme l'a fait la Demanderesse, un matériau thermotrope (pour la première fois) en associant par l'intermédiaire de liaisons hydrogène deux espèces de départ qui ne sont pas mésogènes quand elles sont prises à l'état séparé.

La présente invention concerne donc de nouveaux matériaux thermotropes caractérisés en ce qu'ils consistent en des adducts répondant à la formule générale :

$$(A)_p - \Sigma \;---\; \overline{\Sigma} - (B)_q \qquad (I)$$

dans laquelle :

- les ensembles $(A)_p - \Sigma$ --- $\overline{\Sigma} - (B)_q$, quand ils sont pris isolément,

représentent les structures des réactifs de départ consistant dans des espèces non mésogènes ;

- le symbole $\Sigma$ représente un groupe fonctionnel, dérivant d'une molécule ou d'un groupe de molécules, qui est capable de former au moins deux liaisons hydrogène avec le groupe fonctionnel complémentaire représenté par le symbole $\overline{\Sigma}$ dérivant lui aussi d'une molécule ou d'un groupe de molécules ;
- les symboles A et B, qui peuvent être identiques ou différents, représentent les substitutions portées par les groupes fonctionnels $\Sigma$ et $\overline{\Sigma}$ et consistent dans de longues chaînes hydrocarbonées, linéaires ou ramifiées, pouvant renfermer un ou plusieurs hétéroatome(s), dont la longueur, qui contribue en combinaison avec la structure du motif central $\Sigma$ --- $\overline{\Sigma}$ à induire la propriété thermotrope au moment de l'association des

ensembles $(A)_p - \Sigma$ et $\overline{\Sigma} - (B)_q$, est ajustée à la valeur appropriée ;

- les symboles p et q représentent des nombres entiers, identiques ou différents, égaux à 1 ou 2.

Dans le motif central de structure $\Sigma$ --- $\overline{\Sigma}$, le symbole "---" compris entre $\Sigma$ et $\overline{\Sigma}$ représente au moins deux liaisons hydrogène.

Une liaison hydrogène est formée habituellement entre un donneur de proton et un accepteur de proton et elle peut être illustrée par exemple

par la formule $>NH --- O = C<$ où $>NH$ et $O = C<$ constituent des sites

complémentaires.

Les structures des molécules ou qroupes de molécules desquels dérivent les groupes fonctionnels complémentaires $\Sigma$ et $\overline{\Sigma}$ ne sont pas particulièrement critiques dès l'instant où :

(i) l'ensemble de ces structures apporte au moins deux paires de sites complémentaires et où les sites complémentaires donneur et accepteur d'une même paire sont capables de se situer sensiblement dans un même plan et de prendre une orientation correcte,

et (2i) lesdites structures permettent l'installation des substitutions A et B adéquates.

Comme exemples de sites donneurs et accepteurs, on citera : les

sites donneurs >N - H, $-NH_2$, -O-H et les sites accepteurs >C = O, -O-, =N, >N-.

Dans le cadre de la présente invention, les molécules ou groupes de molécules desquels dérivent les qroupes fonctionnels Σ et $\bar{\Sigma}$, qui doivent comprendre chacun au moins deux sites donneur(s) et/ou accepteur(s), correspondent généralement à des composés monocycliques contenant un ou plusieurs hétéroatome(s) tels que notamment N, O, S ou à des composés polycycliques, ortho- ou péricondensés, contenant également un ou plusieurs hétéroatome(s) tels que notamment N, O, S. Les sites donneurs et accepteurs capables de former des liaisons hydrogène peuvent (i) être contenus dans le (ou les) cycle(s) et/ou (2i) constituer une (ou plusieurs) substitution(s) portée(s) par ce (ou ces) cycle(s).

Des exemples de couples de composés cycliques complémentaires qui peuvent servir à confectionner les réactifs de départ, puis le motif central $\Sigma — \bar{\Sigma}$ des adducts selon l'invention sont notamment : le couple cytosine (ou oxy-2 amino-4 pyrimidine)/isocytosine (ou amino-2 hydroxy-4 pyrimidine) ; le couple guanine (ou amino-2 oxy-6 purine)/cytosine ; le couple adénine (ou amino-6 purine)/uracil (ou dioxy-2,4 pyrimidine) ; le couple dialkanamido-2,6 pyridine/uracil. Conviennent encore les couples différents des couples précités ou leurs analogues qui sont décrits dans les articles suivants :

– B. FEIBUSH et al., J. Am. Chem. Soc. 1986, 108, 3310-3318, comme par exemple le couple dialkanamido-2,6 pyridine/thymine (ou méthyl-5 dioxy-2,4 pyrimidine), et
– R. KELLY et al., J. Am. Chem. Soc. 1987, 109, 6549-6551.

Comme adducts thermotropes préférentiellement représentatifs de la présente invention, on peut citer les adducts de formule (I) dans la structure desquels :

– le motif central $\Sigma — \bar{\Sigma}$ dérive notamment des couples de composés hétérocycliques complémentaires suivants : C1 = cytosine/isocytosine, C2 = guanine/cytosine, C3 = adénine/uracil, C4 = dialkanamido-2,6 pyridine/uracil, C5 = dialkanamido-2,6 pyridine/thymine,
– et les substitutions A et B sont situées en une position opposée ou sensiblement opposée par rapport à celle occupée par les sites donneur(s) et/ou accepteur(s).

Comme adducts thermotropes encore plus préférentiellement représentatifs de la présente invention, on peut citer les adducts de formule (I) dans la structure desquels :

– le motif central $\Sigma - \bar{\Sigma}$ dérive des couples de composés hétérocycliques complémentaires C1 à C5 précités ;
– les substitutions A et B, identiques ou différentes, situées en position opposée ou sensiblement opposée par rapport à celle occupée par les sites donneur(s) et/ou accepteur(s), représentent chacune une longue chaîne aliphatique hydrocarbonée, pouvant renfermer un ou plusieurs hétéroatome(s) tels que notamment N, O, S, qui est ramifiée et répond aux conditions supplémentaires suivantes :
    - la chaîne principale de chaque chaîne ramifiée des substituants A et B comporte un nombre total d'atomes n1 se situant dans l'intervalle allant de 15 à 25,
    - ladite chaîne principale possède une chaîne secondaire comportant un nombre total d'atomes n2 se situant dans l'intervalle allant de 11 à 20,
    - la somme des nombres d'atomes n1 + n2 au niveau de chacun des substituants A et B est au moins égal à 28 atomes ; et
– les symboles p et q sont égaux à 1.

Il doit être entendu que les atomes qui sont pris en compte dans la détermination des nombres n1 et n2 sont les atomes de carbone plus éventuellement les hétéroatomes qui constituent la chaîne (principale ou secondaire) considérée ; par exemple un enchaînement du type : $- CO - O - CH_2 - CH_2 - CH_2 - O -$ comporte 6 atomes et un enchaînement du type : $- CH_2 - NH - CO -$ comporte 3 atomes.

Comme exemples spécifiques d'adducts thermotropes appartenant au groupe des adducts qui sont "encore plus préférentiellement représentatifs de la présente invention", on peut citer notamment les composés de formule :

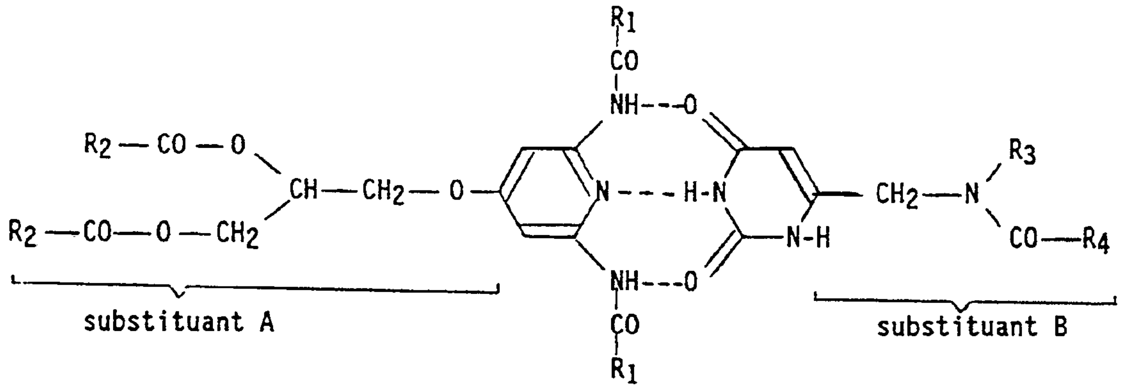

(II)

dans laquelle :

– le symbole $R_1$ représente un radical alkyle linéaire ayant de 1 à 4 atomes de carbone ;

– les symboles $R_2$ et $R_4$, identiques ou différents, représentent chacun un radical alkyle linéaire ayant de 11 à 18 atomes de carbone ;

– le symbole $R_3$, identique à ou différent de $R_2$ et $R_4$, représente un radical alkyle linéaire ayant de 12 à 20 atomes de carbone ;

– avec la condition supplémentaire selon laquelle la somme des atomes de carbone contenus dans l'ensemble $R_3$+$R_4$ du substituant B est égale au moins à 25.

Les adducts thermotropes selon la présente invention peuvent être préparés en réalisant un mélange homogène des réactifs précurseurs $(A)_{\overline{p}}\!-\!\!-\Sigma$ et $\overline{\Sigma}\!-\!\!-(B)_q$ utilisés en quantités équimolaires. Selon les caractéristiques physiques des ingrédients, cette opération peut consister à appliquer les techniques usuelles pour le mélange de solides finement divisés ou bien à effectuer une solution ou une suspension de l'un des constituants du mélange dans l'autre, maintenu à l'état liquide sous l'effet de la température et/ou par emploi d'un milieu solvant.

De préférence, on opère en présence d'un solvant polaire aprotique ou d'un mélange de pareils solvants commun(s) aux réactifs et adduct souhaité en opérant à une température allant de la température ambiante à la température d'ébullition du milieu solvant choisi ; les solvants préférés sont notamment le dioxanne, le tétrahydrofuranne, le chlorure de méthylène, le tétrachlorure de carbone. Dans ces conditions, pour isoler l'adduct, on peut par exemple le faire précipiter par addition dans la solution réactionnelle d'un non-solvant ou d'un mélange de non-solvants et séparer l'adduct précipité, mais il est aussi possible d'obtenir l'adduct par élimination du (ou des) solvant(s) de la solution réactionnelle par exemple par distillation sous pression réduite.

Quand la préparation qui vient d'être décrite se fait en milieu solvant, on doit considérer que la structure de l'adduct souhaité a atteint sa cohésion maximale une fois l'adduct complètement séparé du (ou des) solvant(s) mis en oeuvre.

Les réactifs $(A)_{\overline{p}}\!-\!\!-\Sigma$ et $\overline{\Sigma}\!-\!\!-(B)_q$ dont on part sont des composés qui peuvent être préparés à partir de matières premières disponibles en appliquant des méthodes de synthèses connues de l'homme de métier.

Par exemple, dans le cas des adducts thermotropes de formule (II), le réactif A——Σ ayant la structure 3 suivante :

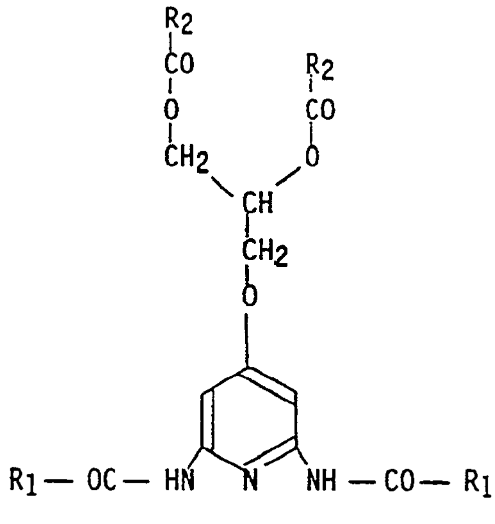

3

où les symboles $R_1$ et $R_2$ ont les significations données ci-avant à propos de la formule (II), correspond à des espèces qui sont nouvelles et peuvent être obtenues à partir d'allyloxy-4 diamino-2,6 pyridine 1 (cf. D.G. MARKEES et al., J. Am. Chem. Soc., 1956, 78, 4130) en appliquant le schéma de synthèses suivant :

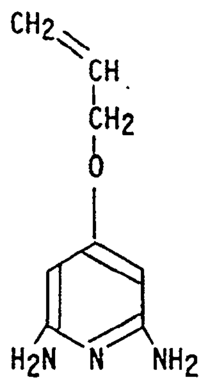

1

1

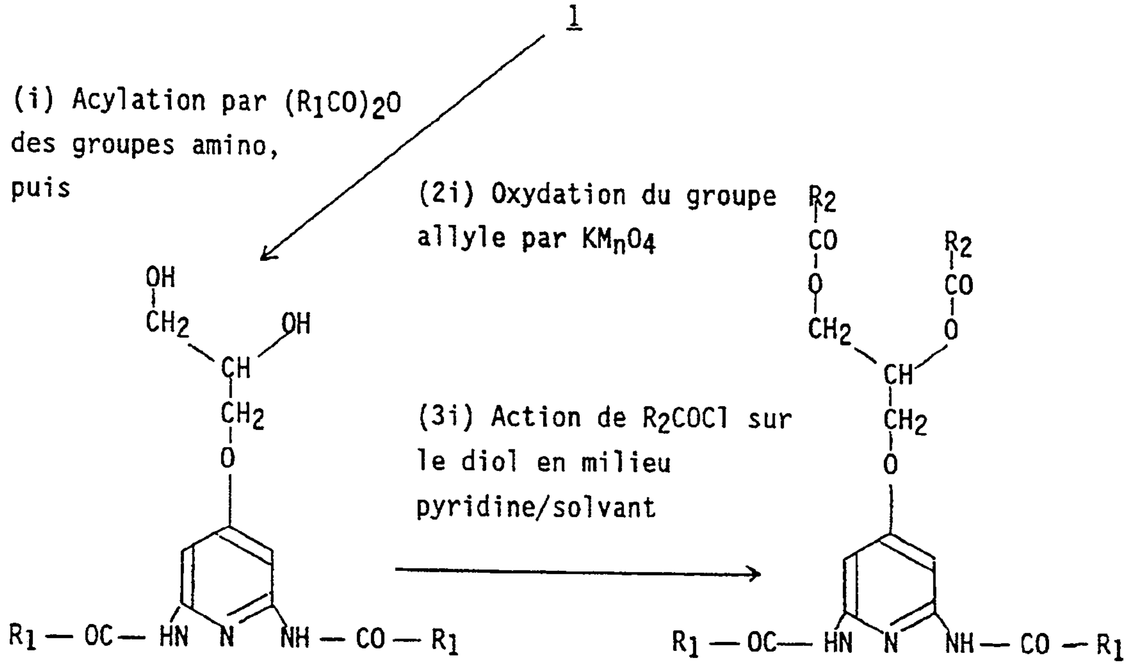


2 3

S'agissant du réactif $\overline{\Sigma}$—B ayant la structure 7 suivante :

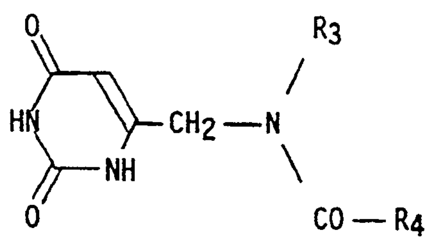

7

où les symboles $R_3$ et $R_4$ ont les significations données ci-avant à propos de la formule (II), il correspond là aussi à des espèces qui sont nouvelles et peuvent être obtenues à partir de formyl-6 uracil 4 (cf. T.B. JOHNSON et al., J. Am. Chem. Soc., 1931, 53, 1989) ou de chlorométhyl-6 uracil 6 (cf. I. VON KLOSA, Arzneim. Forsch./Drug Res., 1980, 30, 228) en appliquant le schéma de synthèse suivant :

Voie a :

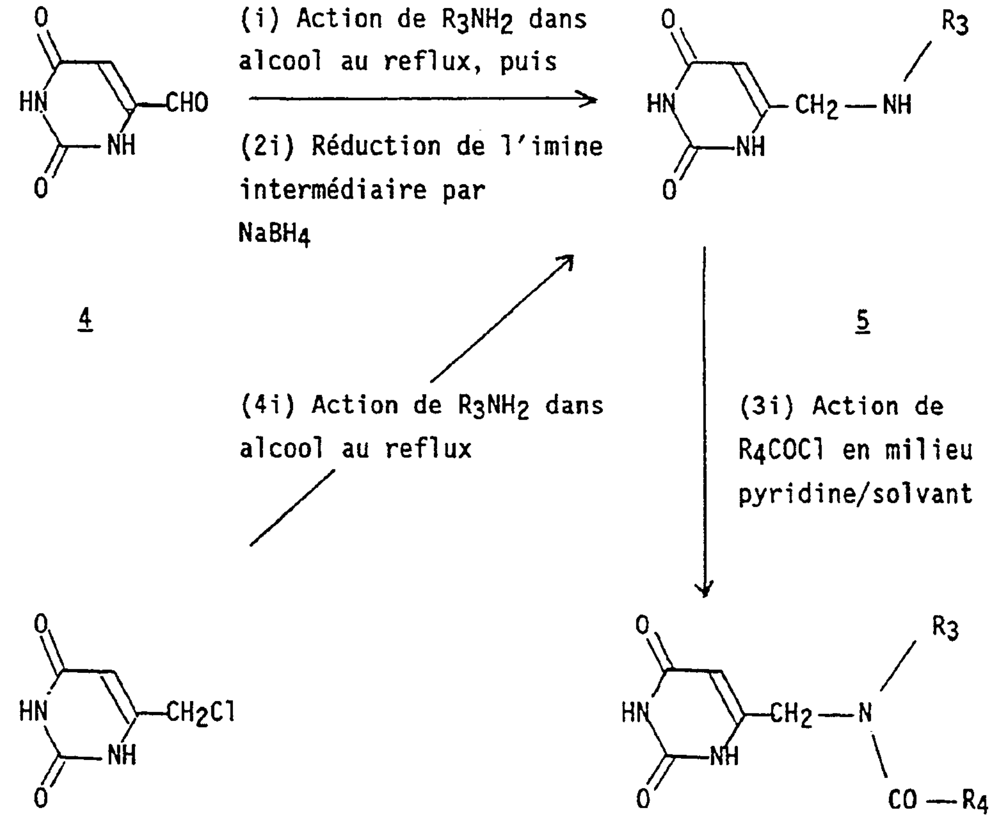


4

5

Voie b :

6

7

Les adducts thermotropes selon la présente invention possèdent la qualité de former des phases de cristal liquide (ou mésophase) à de basses températures comprises par exemple entre 50°C et 80°C. La position par rapport à la température ambiante (23°C) et l'étendue en températures de la plage d'anisotropie peuvent être ajustées en jouant sur la structure des chaînes A et B et sur celle des groupes fonctionnels $\Sigma$ et $\overline{\Sigma}$. Les adducts selon l'invention peuvent servir comme cristaux liquides par exemple dans des dispositifs d'affichage d'informations ou des dispositifs modificateurs de lumière d'appareils électroniques et optiques. A noter encore que le

réactif de formule $(A)_p - \Sigma$ ou $\overline{\Sigma} - (B)_q$ peut être incorporé dans des

substrats organisés tels que des films moléculaires ou des agrégats du genre micelles dans le but de constituer, grâce à l'incorporation ultérieure du réactif complémentaire et à la formation subséquente in situ d'un adduct thermotrope conforme à l'invention (localisable par exemple par des moyens optiques), un site reconnaissable dans ce substrat qui pourra être traité ensuite sélectivement en vue d'obtenir des propriétés nouvelles.

Les exemples qui suivent illustrent de manière non limitative comment la présente invention peut être mise en pratique.

Dans ces exemples, les adducts thermotropes conformes à la présente invention, ainsi que les réactifs de départ qui leur donnent naissance, ont été identifiés par différentes analyses concernant la détermination des caractéristiques de la structure chimique (infra-rouge ; résonance magnétique nucléaire du proton et du carbone 13 ; analyse élémentaire). Les caractéristiques des propriétés thermotropes ont été déterminées : par analyse calorimétrique différentielle (DSC) à l'aide d'un appareil PERKIN-ELMER DSC 2 équipé d'un calculateur HEWLETT-PACKARD HP 86 pour l'acquisition et le traitement des données ; par des observations au moyen d'un microscope polarisant couplé à une platine chauffante qui permet d'identifier la nature des phases

en présence ; et par des examens par diffraction des rayons X (RX).

EXEMPLES 1 A 5 :

On décrit dans ces exemples cinq adducts thermotropes conformes à la présente invention qui répondent à la formule :

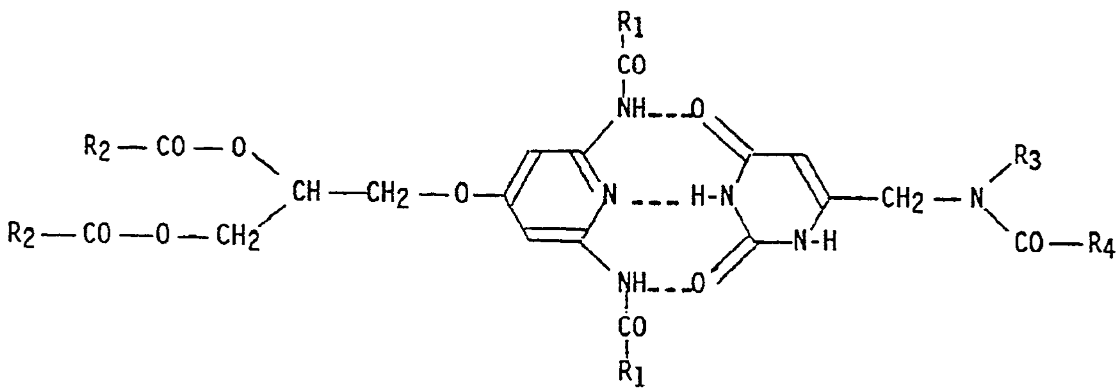

(II)

Réactif précurseur A — Σ de structure 3

Réactif précurseur $\bar{\Sigma}$ — B de structure 7

Le tableau 1 suivant rassemble, exemple par exemple, le données touchant à la substitution des réactifs précurseurs et des adducts obtenus :

TABLEAU 1

| EXEMPLE | DESIGNATION ADDUCT | REACTIFS PRECURSEURS | | SUBSTITUTIONS | | | |
|---|---|---|---|---|---|---|---|
| | | A — Σ | $\bar{\Sigma}$ — B | A — Σ | | $\bar{\Sigma}$ — B | |
| | | | | $R_1$ | $R_2$ | $R_3$ | $R_4$ |
| 1 | 3a/7e | 3a | 7e | $CH_3$ | $C_{11}H_{23}$ | $C_{12}H_{25}$ | $C_{17}H_{35}$ |
| 2 | 3b/7e | 3b | 7e | $CH_3$ | $C_{17}H_{35}$ | $C_{12}H_{25}$ | $C_{17}H_{35}$ |
| 3 | 3C/7e | 3c | 7e | n-$C_4H_9$ | $C_{17}H_{35}$ | $C_{12}H_{25}$ | $C_{17}H_{35}$ |
| 4 | 3b/7f | 3b | 7f | $CH_3$ | $C_{17}H_{35}$ | $C_{16}H_{33}$ | $C_{17}H_{35}$ |
| 5 | 3b/7g | 3b | 7g | $CH_3$ | $C_{17}H_{35}$ | $C_{18}H_{37}$ | $C_{11}H_{23}$ |

A titre d'essais comparatifs, on décrit cinq adducts correspondant à la formule (II) mais qui ne sont pas thermotropes et ne font pas partie en conséquence de la présente invention ; le tableau 2 suivant rassemble essai par essai les données touchant à la substitution de ces adducts :

TABLEAU 2

| ESSAI | DESIGNATION ADDUCT | REACTIFS PRECURSEURS | | SUBSTITUTIONS | | | |
|---|---|---|---|---|---|---|---|
| | | A— Σ | $\overline{\Sigma}$— B | A— Σ | | $\overline{\Sigma}$—B | |
| | | | | $R_1$ | $R_2$ | $R_3$ | $R_4$ |
| A | 3b/7a | 3b | 7a | $CH_3$ | $C_{17}H_{35}$ | $C_8H_{17}$ | $C_7H_{15}$ |
| B | 3b/7b | 3b | 7b | $CH_3$ | $C_{17}H_{35}$ | $C_8H_{17}$ | $C_{17}H_{35}$ |
| C | 3b/7c | 3b | 7c | $CH_3$ | $C_{17}H_{35}$ | $C_{12}H_{25}$ | $C_7H_{15}$ |
| D | 3b/7d | 3b | 7d | $CH_3$ | $C_{17}H_{35}$ | $C_{12}H_{25}$ | $C_{11}H_{23}$ |
| E | 3b/7e | 3d | 7e | i-$C_3H_7$ | $C_{17}H_{35}$ | $C_{12}H_{25}$ | $C_{17}H_{35}$ |

1. Synthèse des dérivés de la 2,6-diaminopyridine 3a-d :

4-allyloxy-2,6-bis-(N-acétylamino)pyridine :

On mélange à température ambiante 975 mg (5,9 mmol) de 4-allyloxy-2,6-diaminopyridine 1, dans 10 ml de pyridine. Après une nuit on verse dans l'eau et extrait à l'éther éthylique. La phase organique est lavée à l'eau puis avec une solution saturée de sel. Après évaporation de l'éther, le résidu (1,4 g) est filtré sur une colonne de 50 g d'alumine en éluant avec $CH_2Cl_2$. Le produit obtenu (1,25 g, rdt 85%), pur d'après la CCM (éluant : EtOH-$CH_2Cl_2$2 : 98), est utilisé tel quel dans l'étape suivante.

4-allyloxy-2,6-bis-(N-valérylamino)pyridine :

Préparé comme précédemment par réaction de 1 avec l'anhydride valérique.

4-allyloxy-2,6-bis-(N-isobutyrylamino)pyridine :

Préparé comme précédemment par réaction de 330 mg (2 mmol) de 1 avec 1,26 g (8 mmol) d'anhydride isobutyrique dans 3 ml de pyridine. Rendement 95%. F=97°C.

2,6-bis-(N-acétylamino)-4-(2,3-dihydroxy)-propoxypyridine ; structure 2 avec $R_1$ = $CH_3$, dite 2a :

A une solution de 1,7 g (6,8 mmol) de 4-allyloxy-2,6-bis-(N-acétylamino)pyridine dans 50 ml d'acétone refroidie à 10-20°C, on ajoute par portions 80-100 ml d'une solution 0,1 M de $KMnO_4$ (durée de l'addition 2 heures). L'excès de $KMnO_4$ est détruit par addition de $Na_2SO_3$ et le mélange réactionnel filtré sur célite. Le filtrat est évaporé sous vide et le résidu dissous dans un mélange $CHCl_3$-MeOH 9 : 1 et chromatographié sur 50 g de silice en éluant avec le même solvant. On récupère en tête 0,3 g de produit puis 1 g de diol 2 (rendement 63% compte tenu du produit de départ récupéré).

2,6-bis-(N-valérylamino)-4(2,3-dihydroxy)-propoxypyridine ; structure 2 avec $R_1$ = n-$C_4H_9$, dite 2b :

Préparé comme 2a à partir de 4-allyloxy-2,6-bis-(N-valérylamino) pyridine. Après chromatographie du produit brut sur silice, on obtient 2b avec un rendement de 65%.

2,6-bis-(N-isobutyrylamino)-4-(2,3-dihydroxy)-propoxypyridine ; structure 2 avec $R_1$ = i-$C_3H_7$ dite 2c :

Préparé comme 2a à partir de 550 mg (1,8 mmol) de 4-allyloxy-2,6-bis-(N-isobutyrylamino)pyridine. Après chromatographie du produit brut (700 mg) sur 25 g de silice, on obtient 440 mg (rendement 65%). Pour analyses, on recristallise dans un mélange EtOH à 95%-$CHCl_3$-éther. Le produit est solvaté. Après désolvatation, F = 134°C.

Distéarate de 2,6-bis-(N-acétylamino)-4-(2,3-dihydroxy)-propoxypyridine 3b :

A une solution de 480 g de diol 2a (1,7 mmol) dans 100 ml de $CHCl_3$ distillé sur $P_2O_5$ et 5 ml de pyridine, refroidie à 0°C, on ajoute en 30 minutes et sous agitation une solution de 1,5 ml (4,44 mmol) de chlorure de stéaroyle dans 50 ml de $CHCl_3$ distillé sur $P_2O_5$. Après 24 heures d'agitation on lave la phase organique successivement à l'eau, avec HCl dilué, à l'eau, avec une solution aqueuse de $Na_2CO_3$ à 2% puis à l'eau, sèche sur $MgSO_4$ et évapore sous vide. Le résidu est chromatographié sur silice en éluant avec $CHCl_3$-méthanol 98: 2. On obtient 990 mg d'ester pur, rendement 71%. Le produit peut être recristallisé dans un mélange $CH_2Cl_2$-MeOH par évaporation lente de $CH_2Cl_2$. Les propriétés physiques (RMN, points de fusion, analyses) sont rassemblées dans les tableaux 3, 4 et la figure 1.

Dilaurate de 2,6-bis-(N-acétylamino)-4-(2,3-dihydroxy)-propoxypyridine 3a :

Distéarate de 2,6-bis-(N-valérylamino)-4-(2,3-dihydroxy)-propoxypyridine 3c :

Distéarate de 2,6-bis-(N-isobutyrylamino)-4-(2,3-dihydroxy)-propoxypyridine 3d :

Préparés comme 3b à partir des diols 2a, 2b, 2c respectivement et des chlorures d'acide appropriés. Les propriétés physiques (RMN, points de fusion, analyses) sont rassemblées dans les tableaux 3 et 4 et la figure 1.

2. Synthèse des dérivés de l'uracil 7e-g :

6-(N-hexadécylaminométhyl)uracil ; structure avec $R_3$ = $C_{16}H_{33}$, dite 5c : Voie a.

On chauffe à reflux pendant 4 heures un mélanqe de 241 mg (1 mmol) d'hexadécylamine, 140 mg (1 mmol) de 6-formyluracil dans 50 ml de EtOH à 95%. Après refroidissement le produit qui cristallise est essoré sur buchner puis recristallisé dans l'éthanol et séché sous vide. On obtient 260 mg (0,72 mmol) (rendement 71%) d'imine qu'on traite par 100 mg de $NaBH_4$ dans 70 ml d'EtOH aqueux à 80%. Après 4 heures de reflux sous agitation, on évapore le solvant. On ajoute au résidu 30 ml d'HCl N et essore le chlorhydrate de l'amine. Après recristallisation dans EtOH à 95% on obtient 780 mg (rendement 90%) suffisamment pur pour l'étape suivante.

6-(N-octadécylaminométhyl)uracil ; structure 5 avec R3 = C18H37, dite 5d : Voie b.

On chauffe à reflux et sous agitation pendant 15 heures un mélange de 1,6 g (10 mmol) de 6-chlorouracil commercial et de 5,4 g (20 mmol) d'octadécylamine dans 125 ml d'isopropanol. Après refroidissement on essore l'amine cristallisée qu'on recristallise dans un mélange $CH_2Cl_2$-EtOH. Le produit obtenu, 2,2 g (rendement 56%), est suffisamment pur pour l'étape suivante.

6-(N-octylaminométhyl)uracil ; structure 5 avec $R_3$ = $C_8H_{17}$, dite 5a :

Préparé comme 5d selon la voie b, à partir de 6-chlorouracil et d'octylamine.

6-(N-dodécylaminométhyl)uracil ; structure 5 avec $R_3$ = $C_{12}$ $H_{25}$, dite 5b :

Préparé par les voies a et/ou b.

6-(N-hexadécyl-N-stéaroylaminométhyl)uraci1 7f :

A une suspension de 365 mg (1 mmol) d'amine 5c (ou 402 mg de son chlorhydrate) dans 50 ml de $CHCl_3$ distillé sur $P_2O_5$ et 1 ml de pyridine anhydre, on ajoute en 30 minutes 305 mg (1 mmol) de chlorure de stéaroyle dans 10 ml de $CHCl_3$ distillé sur $P_2O_5$. Après 24 heures d'agitation à température ambiante et sous azote on dilue avec 150 ml de $CHCl_3$ et lave successivement avec HCl N, à l'eau, avec une solution aqueuse de $Na_2CO_3$ à 2% puis à l'eau, sèche sur $MgSO_4$ et évapore sous vide. Le résidu est chromatographié sur silice en éluant avec $CHCl_3$-méthanol 95 : 5. On obtient 490 mg d'amide pur, rendement 78%. Le produit est recristallisé dans un mélange $CH_2Cl_2$-MeOH par évaporation lente de $CH_2Cl_2$. Les propriétés physiques (RMN, points de fusion, analyses) sont rassemblées dans les tableaux 3 et 4 et la figure 2.

Composés 7a, 7b, 7c, 7d, 7e, 7g :

Préparés comme 7f à partir des amines 5a, 5b et 5d. Les propriétés physiques (RMN, points de fusion, analyses) sont rassemblées dans les tableaux 3, 4 et la figure 2.

TABLEAU 3

Propriétés physiques des dérivés 3a-d de la 2,6-diaminopyridine et 7a-g de l'uracil

| Composé | $R_1$ | $R_2$ | $R_3$ | $R_4$ | Transition[a)] T (°C) | Transition[a)] ΔH ($Kcal.mol^{-1}$) |
|---|---|---|---|---|---|---|
| 3a | Me | $C_{11}H_{23}$ | - | - | K 30-46 I[b)] | 12,9 |
| 3b | Me | $C_{17}H_{35}$ | - | - | K 55-58 I | 22,5 |
| 3c | n-Bu | $C_{17}H_{35}$ | - | - | K 37-42 I | 18,3 |
| 3d | i-Pr | $C_{17}H_{35}$ | - | - | K 40-44 I | 18,0 |
| 7a | - | - | $C_8H_{17}$ | $C_7H_{15}$ | K 77 K | 9,5 |
| | | | | | K 148 I | 7,3 |
| 7b | - | - | $C_8H_{17}$ | $C_{17}H_{35}$ | K 82 K | 16,4 |
| | | | | | K 138 I | 7,4 |
| 7c | - | - | $C_{12}H_{25}$ | $C_7H_{15}$ | K 43;75;95 K | 8,1 |
| | | | | | K 143 I | 7,1 |
| 7d | - | - | $C_{12}H_{25}$ | $C_{11}H_{23}$ | K 73-79;88-90 K | 13,0 |
| | | | | | K 142-144 I | 7,9 |
| 7e | - | - | $C_{12}H_{25}$ | $C_{17}H_{35}$ | K 45;70;85 K | 15,4 |
| | | | | | K 135 I | 8,6 |
| 7f | - | - | $C_{16}H_{33}$ | $C_{17}H_{35}$ | K 75-95[b)];98 K | 12,3 |
| | | | | | K 129 I | 5,9 |
| 7g | - | - | $C_{18}H_{37}$ | $C_{11}H_{23}$ | K 55-59 K | 3,3 |
| | | | | | K 100-105 K | 10,7 |
| | | | | | K 132-135 I | 7,1 |

a) Mesures effectuées à l'aide du microcalorimètre Perkin-Elmer DSC2.K = phase cristalline, I = phase isotrope.

b) Transition large correspondant à plusieurs pics mal résolus attribués soit à des transformations K --> K soit à la fusion de plusieurs formes cristallines coexistant dans l'échantillon.

TABLEAU 4

Analyses élémentaires des dérivés 3a-d de la 2,6-diaminopyridine et 7a-g de l'uracil

| Composé | Formule brute | | C% | H% | N% |
|---|---|---|---|---|---|
| 3a | $C_{36}H_{61}N_3O_7$ | Calculé | 66,74 | 9,49 | 6,49 |
| | | Trouvé | | | |
| 3b | $C_{48}H_{85}N_3O_7$ | Calculé | 70,63 | 10,49 | |
| | | Trouvé | 70,8 | 10,3 | |
| 3c | $C_{54}H_{97}N_3O_7$ | Calculé | 72,03 | 10,86 | 4,67 |
| | | Trouvé | | | |
| 3d | $C_{52}H_{93}N_3O_7$ | Calculé | 71,60 | 10,75 | |
| | | Trouvé | 71,6 | 10,7 | |
| 7a | $C_{21}H_{37}N_3O_3$ | Calculé | 66,45 | 9,83 | 11,07 |
| | | Trouvé | 66,5 | 9,8 | 11,2 |
| 7b | $C_{31}H_{57}N_3O_3$ | Calculé | 71,63 | 11,06 | 8,08 |
| | | Trouvé | 71,3 | 11,1 | 8,1 |
| 7c | $C_{25}H_{45}N_3O_3$ | Calculé | 68,92 | 10,41 | 9,65 |
| | | Trouvé | 68,3 | 10,4 | 9,5 |
| 7d | $C_{29}H_{53}N_3O_3$ | Calculé | 70,83 | 10,86 | 8,54 |
| | | Trouvé | | | |
| 7e | $C_{35}H_{65}N_3O_3$ | Calculé | 72,99 | 11,38 | 7,30 |
| | | Trouvé | | | |
| 7f | $C_{39}H_{73}N_3O_3$ | Calculé | 74,11 | 11,64 | |
| | | Trouvé | 74,2 | 11,7 | |
| 7g | $C_{35}H_{65}N_3O_3$ | Calculé | 72,99 | 11,38 | 7,30 |
| | | Trouvé | 73,1 | 11,4 | 7,4 |

FIGURES 1 ET 2

Figure 1. Déplacements chimiques des protons des dérivés de la 2,6-diamino-pyridine 3 (1H RMN ; $CDCl_3$ ; 200 MHz).

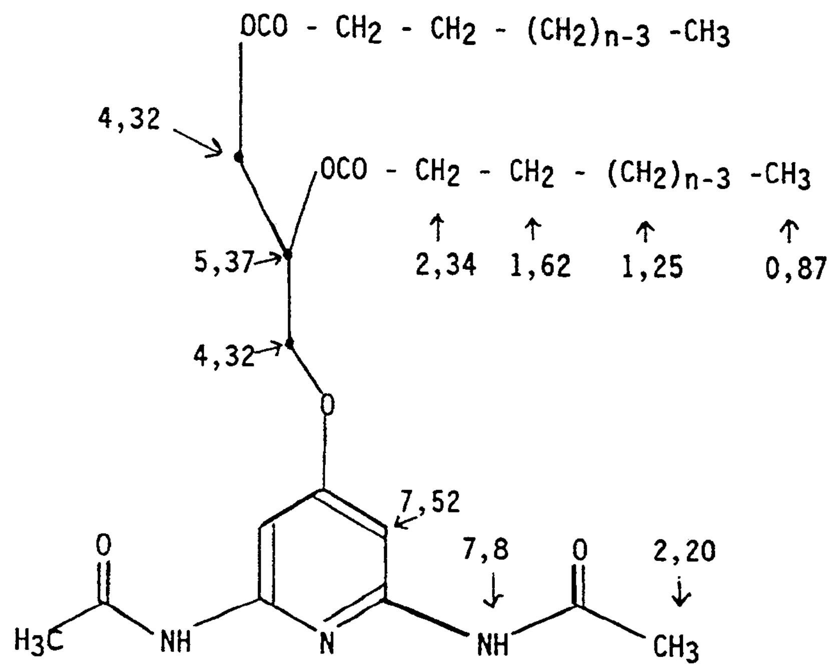

Figure 2. Déplacements chimiques des protons des dérivés de l'uracil 7.

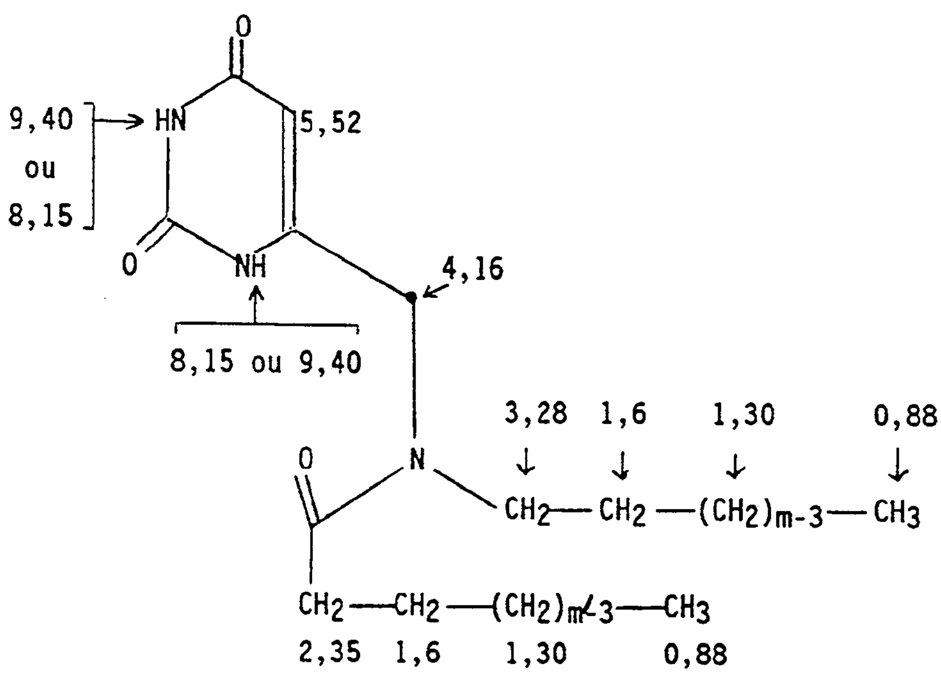

3. Synthèse des adducts conformes et non conformes à l'invention :

Tous les adducts sont préparés chaque fois par pesée de proportions exactement équimolaires des réactifs précurseurs 3 et 7, suivie d'une dissolution dans $CH_2Cl_2$. Après évaporation du solvant, l'adduct obtenu est séché sous vide pendant plusieurs heures.

Les températures et enthalpies de transition mesurées par DSC sont rassemblées dans le tableau 5 suivant. Les échantillons (1 à 3 mg) sont examinés au chauffage puis au refroidissement sur au moins deux cycles (vitesses 5 ou 10°C.min$^{-1}$). Les températures de transition correspondent soit à l'intersection entre la ligne de base et la tangente à la plus grande pente du pic (pic fin) soit au maximum du pic (pic large). Les enthalpies de transition sont déduites de la surface des pics en prenant l'indium comme étalon (enthalpie de fusion 785 cal.mol$^{-1}$).

TABLEAU 5

Températures en °C et enthalpies de transition [$\Delta H$] en kcal.mol$^{-1}$ des adducts des dérivés 3 et 7[a,b].

| Exemple/Essai | Adduct | |
|---|---|---|
| A | 3b/7a | K 30-56;62-82 I |
| B | 3b/7b | K 27-41;62-74 I |
| C | 3b/7c | K 33-49;72-81 I |
| D | 3b/7d | K 30-59;67-87 I |
| 1 | 3a/7e | K 30-46;60-73 (M 71 [0,73]) I |
| 2 | 3b/7e | K 40-45;62-75 (M 72 [0,64]) I |
| 3 | 3c/7e | K 34-39;47-78 (M 57 [1,12]) I |
| E | 3d/7e | K 36-55;62-75 I |
| 4 | 3b/7f | K 30-67;72-82 (M 77 [0,94]) I |
| 5 | 3b/7g | K 20-85 (M 73 [0,60]) 1 |

a) K = phase cristalline, (M) = phase mésomorphe métastable, I = phase isotrope.

b) Lorsque les domaines de température indiqués pour les transitions sont > 10°C, voir Note b) au bas du tableau 3.

Tous les adducts étudiés fondent en-dessous de 90°C. Cinq de ces adducts, ceux conformes aux exemples 1 à 5 de l'invention, présentent une phase mésomorphe métastable confirmée par l'examen au microscope et par RX. Cette phase mésomorphe n'est observée que lorsque les chaînes sont suffisamment longues (7e-g).

L'étude par RMN de l'adduct 3b/7f confirme l'existence de fortes interactions en solution ($CDCL_3$). On observe en effet un fort déplacement vers les champs faibles des signaux NH qui passent de $\delta$ 7,8 (3b) et 8,15; 9,40 (7f) à $\delta$ 9,60 ; 9,90 et 11,5 ppm pour l'adduct, ce qui indique la formation de trois liaisons H. On peut admettre raisonnablement qu'en phase mésomorphe cette structure de complexe ou supermolécule mettant en jeu trois liaisons hydrogène est préservée. L'analyse aux RX des adducts 3a/7e et 3b/7f montre que la mésophase est du type colonnaire hexagonal, avec 4 supermolécules par plateaux distants de 3,5 Å, le diamètre des colonnes étant respectivement de 37,0 et 40,4 Å.

## Revendications

1. Matériaux thermotropes caractérisés en ce qu'ils consistent en des adducts répondant à la formule générale :

$$(A)_p\!\!-\!\!-\Sigma\ \text{---}\ \overline{\Sigma}-\!\!-(B)_q \qquad (I)$$

dans laquelle :

- les ensembles $(A)_p\!-\!\Sigma$ --- $\overline{\Sigma}\!-\!(B)_q$ , quand ils sont pris isolément, représentent les structures des réactifs de départ consistant dans des espèces non mésogènes ;
- le symbole $\Sigma$ représente un groupe fonctionnel, dérivant d'une molécule ou d'un groupe de molécules,

qui est capable de former au moins deux liaisons hydrogène avec le groupe fonctionnel complémentaire représenté par le symbole $\bar{\Sigma}$ dérivant lui aussi d'une molécule ou d'un groupe de molécules ;
– les symboles A et B, qui peuvent être identiques ou différents, représentent les substitutions portées par les groupes fonctionnels $\Sigma$ et $\bar{\Sigma}$ et consistent dans de longues chaînes hydrocarbonées, linéaires ou ramifiées, pouvant renfermer un ou plusieurs hétéroatome(s), dont la longueur, qui contribue en combinaison avec la structure du motif central $\Sigma — \bar{\Sigma}$ à induire la propriété thermotrope au moment de l'association des

ensembles $(A)_p—\Sigma$ et $\bar{\Sigma}—(B)_q$ , est ajustée à la valeur appropriée ;

– les symboles p et q représentent des nombres entiers, identiques ou différents, égaux à 1 ou 2.

2. Adducts selon la revendication 1, caractérisés en ce que les molécules ou groupes de molécules desquels dérivent les groupes fonctionnels $\Sigma$ et $\bar{\Sigma}$, qui doivent comprendre chacun au moins deux sites donneur(s) et/ou accepteur(s), correspondent à des composés monocycliques contenant un ou plusieurs hétéroatome(s) tels que notamment N, O, S ou à des composés polycycliques, ortho- ou péricondensés, contenant également un ou plusieurs hétéroatome(s) tels que notamment N, O, S, les sites donneurs et accepteurs capables de former des liaisons hydrogènes pouvant (i) être contenus dans le (ou les) cycle(s) et/ou (2i) constituer une (ou plusieurs) substitution(s) portée(s) par ce (ou ces) cycles.

3. Adducts selon l'une quelconque des revendications 1 et 2, caractérisés en ce qu'ils correspondent à des composés de formule (I) dans la structure desquels :
– le motif central $\Sigma — \bar{\Sigma}$ dérive des couples de composés hétérocycliques complémentaires suivants : C1 = cytosine/isocytosine, C2 = guanine/cytosine, C3 = adénine/uracil, C4 = dialkanamido-2,6 pyridine/uracil, C5 = dialkanamido-2,6 pyridine/thymine,
– et les substitutions A et B sont situées en une position opposée ou sensiblement opposée par rapport à celle occupée par les sites donneur(s) et/ou accepteur(s).

4. Adducts selon la revendication 3, caractérisés en ce que dans leur structure :
– les substitutions A et B, identiques ou différentes, situées en position opposée ou sensiblement opposée par rapport à celle occupée par les sites donneur(s) et/ou accepteur(s), représentent chacune une longue chaîne aliphatique hydrocarbonée, pouvant renfermer un ou plusieurs hétéroatome(s) tels que notamment N, O, S, qui est ramifiée et répond aux conditions supplémentaires suivantes :
. la chaîne principale de chaque chaîne ramifiée des substituants A et B comporte un nombre total d'atomes n1 se situant dans l'intervalle allant de 15 à 25,
. ladite chaîne principale possède une chaîne secondaire comportant un nombre total d'atomes n2 se situant dans l'intervalle allant de 11 à 20,
. la somme des nombres d'atomes n1 + n2 au niveau de chacun des substituants A et B est au moins égal à 28 atomes ; et
– les symboles p et q sont égaux à 1.

5. Adducts selon la revendication 4, caractérisés en ce qu'ils répondent à la formule :

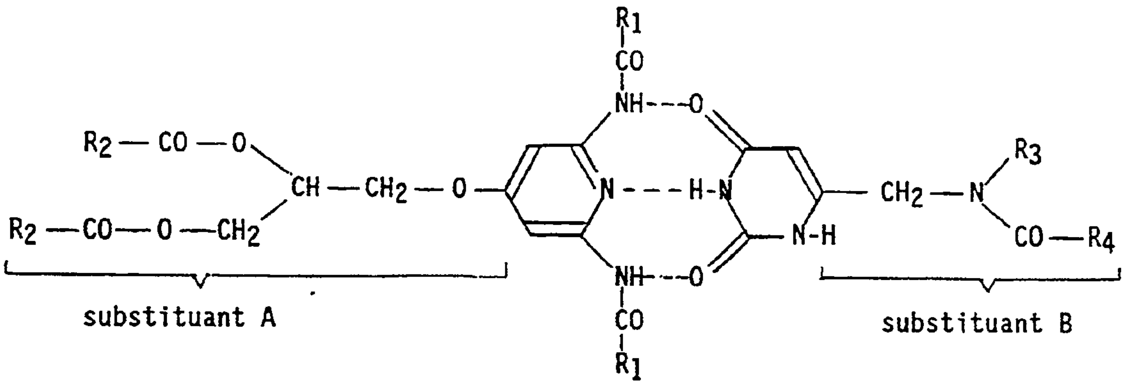

(II)

dans laquelle :

– le symbole $R_1$ représente un radical alkyle linéaire ayant de 1 à 4 atomes de carbone ;

– les symboles $R_2$ et $R_4$, identiques ou différents, représentent chacun un radical alkyle linéaire ayant de 11 à 18 atomes de carbone ;

– le symbole $R_3$, identique à ou différent de $R_2$ et $R_4$, représente un radical alkyle linéaire ayant de 12 à 20 atomes de carbone ;

– avec la condition supplémentaire selon laquelle la somme des atomes de carbone contenus dans l'ensemble $R_3 + R_4$ du substituant B est égale au moins à 25.

6. Procédé de préparation des adducts thermotropes selon l'une quelconque des revendications 1 à 5, caractérisé en ce qu'il consiste à réaliser pour tous moyens connus en soi un mélange homogène des réactifs précurseurs $(A)_p—\Sigma$ et $\overline{\Sigma}—(B)_q$ qui sont engagés en quantités équimolaires.

7. Procédé selon la revendication 6, caractérisé en ce qu'il est conduit en présence d'un solvant polaire aprotique ou d'un mélange de pareils solvants commun(s) aux réactifs précurseurs et adduct souhaité en opérant à une température allant de la température ambiante à la température d'ébullition du milieu solvant choisi.

8. A titre de produits nouveaux spécialement destinés à la mise en oeuvre du procédé selon l'une quelconque des revendications 6 et 7 précédentes, les réactifs précurseurs de formules $(A)_p—\Sigma$ et $\overline{\Sigma}—(B)_q$ dans lesquelles les symboles A, Σ, p, $\overline{\Sigma}$, B et q ont les significations données ci-avant dans l'une quelconque des revendications 1 à 5.

9. Produits selon la revendication 8 de formule $(A)_p—\Sigma$, caractérisés en ce qu'ils possèdent la structure 3 suivante :

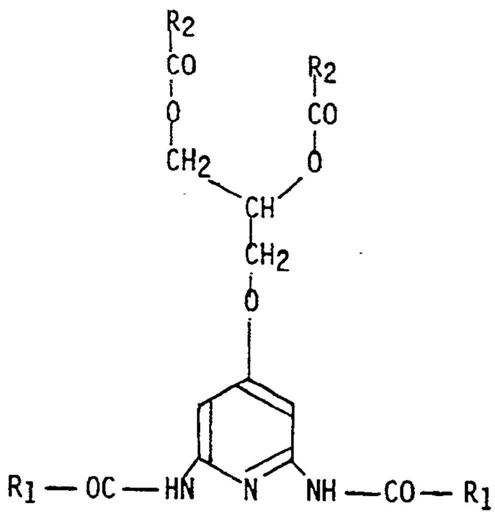

3

où les symboles $R_1$ et $R_2$ ont les significations données ci-avant dans la revendication 5.

**10.** Produits selon la revendication 8 de formule $\overline{\Sigma}$——(B)$_q$, caractérisés en ce qu'ils possèdent la structure 7 suivante :

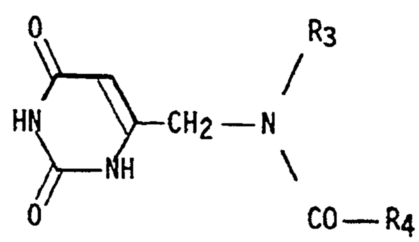

7

où les symboles $R_3$ et $R_4$ ont les significations données ci-avant dans la revendication 5.

Office européen des brevets

**RAPPORT PARTIEL DE RECHERCHE EUROPEENNE**
qui selon la règle 45 de la Convention sur le brevet européen est considéré, aux fins de la procédure ultérieure, comme le rapport de recherche européenne

Numéro de la demande
EP 90 42 0534

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (Int. Cl.4) |
|---|---|---|---|
| A | JOURNAL OF THE AMERICAN CHEMICAL SOCIETY, vol. 109, no. 24, 25 novembre 1987, pages 7531-7533, American Chemical Society, Washington, DC, US; B. FEIBUSH et al.: "HPLC separation of DNA adducts based on hydrogen bonding"<br>* Page 7532, colonne de gauche * | 1,5 | C 07 D 239/54<br>C 07 D 213/75<br>C 07 K 19/34 |
| | -- | | |
| A | JOURNAL OF THE AMERICAN CHEMICAL SOCIETY, vol. 108, no. 12, 11 juin 1986, pages 3310-3318, American Chemical Society, Washington, DC, US; B. FEIBUSH et al.: "Chiral separation of heterocyclic drugs by HLPC: solute-stationary phase base-pair interactions"<br>* Page 3311, figure 3 * ./. | 1,5 | DOMAINES TECHNIQUES RECHERCHES (Int. Cl.4)<br>C 07 D 239/00<br>C 07 D 251/00 |
| | -- | | |

**RECHERCHE INCOMPLETE**

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes: 5,9,10
Revendications ayant fait l'objet de recherches incomplètes: 1-4,6-8
Revendications n'ayant pas fait l'objet de recherches:
Raison pour la limitation de la recherche:

Comme les revendications ne sont pas claires et concises (Art. 84 CBE) et renferment un très grand nombre de produits, une recherche complète n'est pas possible pour des raisons économiques (Directives pour l'Examen à l'OEB, partie B, chapitre III, 3.7).
Donc, la recherche a été basée sur les revendications bien definis et les exemples (Règle 45, Règlement d'Exécution de la CBE).

| Lieu de la recherche | Date d'achèvement de la recherche | Examinateur |
|---|---|---|
| LA HAYE | 27-02-1991 | ENGLISH |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1505.1 03.82

Office européen des brevets

**RAPPORT PARTIEL DE RECHERCHE EUROPEENNE**

Numéro de la demande

EP 90 42 0534
-2-

| DOCUMENTS CONSIDERES COMME PERTINENTS | | | CLASSEMENT DE LA DEMANDE (Int. Cl.4) |
|---|---|---|---|
| Catégorie | Citation du document avec indication, en cas de besoin, des parties pertinentes | Revendication concernée | |
| A | US-A-3 455 942 (R.E. TADESCHI et al.)<br>* Colonne 1, lignes 33-50; colonnes 2-3, exemple 3; colonne 6, lignes 72-75 *<br>-- | 8,9 | |
| A | DE-A-2 729 080 (J. KLOSA)<br>* Revendication; page 2 *<br>-- | 8,10 | **DOMAINES TECHNIQUES RECHERCHES (Int. Cl.4)** |
| P,X | JOURNAL OF THE CHEMICAL SOCIETY, CHEMICAL COMMUNICATIONS, no. 24, 15 décembre 1989, pages 1868-1870, Letchworth, GB; M.-J. BRIENNE et al.: "Macroscopic expression of molecular recognition. Supramolecular liquid crystalline phases induced by association of complementary heterocylic components"<br>* En entier *<br>---- | 1-10 | |

OEB Form 1505.3 06.78